# EUROPEAN PATENT SPECIFICATION

(11) **EP 2 301 621 B1**
(45) Date of publication and mention of the grant of the patent: **20.11.2013**
(21) Application number: 09171271.1
(22) Date of filing: 24.09.2009
(51) Int. Cl.: A61M 39/10, A61M 25/00

(54) **Connection device for medical tubing**
Verbindungsvorrichtung für medizinische Schläuche
Dispositif de raccordement pour tubulure médicale

(43) Date of publication of application: 30.03.2011
(73) Proprietor: Dentsply IH AB, 431 21 Mölndal (SE)
(72) Inventor: Saltell, Josef, 412 62, GÖTEBORG (SE); Andréen, Erik, 41763 Göteborg (SE)
(74) Representative: Lind, Urban Arvid Oskar

(56) References cited:
- EP-A- 0 661 075
- EP-A- 1 181 946
- WO-A-2006/091952
- FR-A- 2 714 295
- US-A- 5 279 597
- US-A- 5 556 387
- US-A1- 2005 192 559

## Description

### Field of the invention

The present invention generally relates to a coupling device for releasable connection of medical tubing, such as catheters, of the type where no coupling parts are introduced into the lumen of the medical tubing.

### Background of the invention

Medical tubings, such as catheters, are used in various medical applications. In principle, catheters are used either to remove fluid from a patient, e.g. for draining of blood etc. from wounds, or for providing fluids to the patient, e.g. for administering medicaments. For example, small catheters - typically an outer diameter size of about 2 mm is suitable - can be used for administering liquid medicaments to a patient for post-operative pain relief.

In use, the catheters normally need to be connected, both mechanically and fluidly, to other medical devices, such as a drug administration device. The connection to the medical tubing needs to be adequately strong, reliable and fluid tight, but it is also of importance that the flow capacity of the medical tubing is not significantly restricted. Further, the medical tubing connection operation should preferably be simple and quick. Still further, the connection should preferably be releasable, making it possible both to disconnect and reconnect the medical tubing.

A common type of coupling devices for medical tubing uses one part which is inserted into the lumen of the medical tubing, and another part which clamps the end part of the medical tubing between the inserted part and an outer part. An example of such a coupling device is disclosed in US 6971390. However, this type of connectors has certain drawbacks. For example, arrangement of a part inside the medical tubing is relatively complicated, especially for small medical tubings, which makes the coupling process tedious and cumbersome. Further, the introduction of a part into the medical tubing lumen often leads to a restriction in the flow capacity of the medical tubing, and/or a requirement that the medical tubing is made of a flexible and elastic material. Still further, most previously known connector devices are also relatively complicated and expensive to produce.

WO 2006/091952 discloses a coupling device in accordance with the preamble of claim 1. US 2005/0192559 discloses a similar type of coupling device in which a clamp is used to compress a medical tubing. EP 1 181 946 discloses complicated and structurally different connection arrangement.

There is therefore a need for a coupling device of a type without any part protruding into the lumen of the medical tubing, and which provides a quick and reliable connection of the medical tubing end, and which can be produced to a relatively low cost.

### Summary of the invention

It is a general object of the present invention to alleviate the above-discussed problems, and provide a coupling device for medical tubing providing a reliable connection without any parts being inserted into the lumen of the medical tubing.

According to the invention, there is provided a coupling device for releasable connection of a medical tubing in accordance with claim 1.

Preferably, the relatively uniform reduction of the inward diameter of the intermediate part is arranged to occur over a length being at least two times the outer diameter of the medical tubing, and preferably at least three times said diameter, and most preferably at least five times said diameter.

By means of this coupling device, a very strong, reliable and fluid tight connection is achievable. Since the compression on the medical tubing is applied relatively uniformly and over an extended length, a very good holding engagement is obtainable, without any significant change in the medical tubing properties. Thus, the flow of the medical tubing is essentially not affected at all when using the new coupling device.

Preferably, the engagement between the coupling device and the medical tubing is obtained through a friction fit. However, the alternatively or additionally, the engagement may also be a mechanical engagement, such as a mechanical interlocking. This may e.g. be obtained through a surface structure provided on either the inwardly facing coupling surface of the coupling device, or the outwardly facing surface of the medical tubing, or a combination of the two. The surface structure may e.g. comprise a roughened surface, and preferably in a certain pattern, such as grooves, protruding ribs or the like. The surface structure may also form barb-like protrusions. Further, the surface structure may comprise holes or perforations, e.g. arranged through the wall of the medical tubing. The surface structure of the end of the medical tubing and the coupling device may also correspond and match, in order to obtain a good engagement and interlocking between the parts.

The new coupling device is relatively simple and cost-efficient to produce, since if comprises few parts, all of which are producible with straight-forward production methods, such as by injection molding.

Further, the new coupling device is very fast and simple to use. The medical tubing end need only to be inserted through the first end of the hollow body member, and the compression means is moved into a position so that a compression force is applied to the medical tubing through the intermediate part of the hollow body member. Dismounting of the medical tubing is just as easy, and the medical tubing can easily be disconnected and reconnected over and over again, without damaging the medical tubing.

Still further, the present coupling device is useable for essentially all types of medical tubing, such as catheters, regardless of the size and material of the medical tubing.

However, the present invention is particularly useful for small medical tubings, such as medical tubings having an outer diameter in the range 0.5-3.0 mm, and preferably in the range 1.0 - 2.5 mm, and most preferably about 2 mm.

In particular, the present coupling device is highly suitable for connection of soft and pliable medical tubing, which tend to be deformed with previously known connection arrangements.

Further, by avoiding the introduction of any material into the lumen of the medical tubing, the risk for contamination is greatly reduced. By means of the present invention, the coupling device is only in contact with the outer surface of the medical tubing. Further, the connection of the medical tubing to the coupling device can be performed without touching the end of the medical tubing, or any part in the vicinity of the end, which also leads to a reduced risk of contamination.

The intermediate part preferably comprises a plurality of fingers, wherein the fingers are to at least some extent spaced apart in a circumferential direction. Hereby, the fingers are easily moveable inwardly and outwardly, without restricting each other. However, alternatively a solid intermediate part, e.g. made of an elastic material, is also feasible.

The compression means comprises a hollow compression sleeve being arrangeable over the intermediate part of the hollow body member, and being engageable with an engagement area of said hollow body member arranged, whereby engagement between the hollow compression sleeve and the hollow body member forces the hollow compression sleeve to exert a force towards the intermediate part.

The engagement between the hollow compression sleeve and the hollow body member occurs through a screw threading. Hereby, longitudinal displacement between the compression sleeve and the hollow body member becomes very easy and controllable by applying a rotational movement between the compression sleeve and the hollow body member.

The engagement area of the hollow body is arranged between the intermediate part and the second end.

The intermediate part of the hollow body member comprises at least one surface tapering in the direction of the first end, and the hollow compression sleeve comprises a corresponding at least one tapering surface, whereby movement of the hollow compression sleeve over said hollow body member in a direction from said first end to said second end provides an engagement between said tapering surfaces, and thereby a compressing force on said intermediate part.

According to one embodiment, the intermediate part comprises one conically tapering part, and the compression sleeve comprises one or several correspondingly tapering surfaces. In case the above-discussed alternative with an intermediate part having a plurality of arms is used, the conically tapering part would in this case be divided into a corresponding number of segments.

According to another embodiment, the intermediate part comprises a plurality of outwardly protruding parts, said parts forming a plurality of tapering surfaces. Preferably, the protruding parts are formed as a screw threading, and whereby the at least one tapering surface of the hollow body member forms a corresponding screw threading.

In case both the intermediate part and the engagement area of the hollow body member engages with the compression sleeve through screw threading, the screw pitch of the screw threading between the hollow compression sleeve and the engagement area of the hollow body member is preferably greater than the screw pitch of the screw threading between hollow compression sleeve and the intermediate part of the hollow body member. Hereby, the screw threading of the engagement area forces the screw threading of the compression sleeve to "climb" onto the screw threading of the intermediate part, due to the tapering surfaces, thereby causing the intermediate part to be compressed inwardly.

By means of the screw threading between the hollow compression sleeve and the hollow body member, a relatively small movement in the rotational direction is transferred to a relatively small axial movement, which works on the entire engagement area, and provides engagement over a relatively large axial extension of the coupling device.

Preferably, the compression sleeve is lockable on the hollow body when it arrives at an engaged and coupled state. This locking may e.g. be achieved by a snap locking arrangement which automatically locks the parts at a certain point of engagement.

The hollow body member is further preferably provided with a shoulder forming a positive stop for the hollow compression sleeve, prohibiting continued engagement between the hollow compression sleeve and the hollow body member at a certain point, and thereby providing a maximum for the reduction of the inward diameter of the intermediate part. Hereby, the risk of over-tightening the connection, which could possibly lead to damage of the medical tubing, is prevented.

The coupling device also preferably further comprises a tube of a flexible material, arranged in the hollow body member in the area of the intermediate part, whereby the tube is situated between the intermediate part of the body member and a medical tubing end inserted into the coupling device. Hereby, the friction between the medical tubing and the coupling device is improved, and also, it becomes easier to obtain a tight and leak proof connection.

These and other aspects of the inventive concept will be apparent from and elicited with reference to the embodiments described hereinafter.

### Brief description of the drawings

By way of example embodiments of the invention will now be described with reference to the accompanying drawings in which:
Fig. 1 is a cross-sectional view of a first embodiment of a coupling device according to the invention in an unconnected disposition;
Fig. 2 is a side-view of the coupling device of Fig. 1;
Fig. 3 is a cross-sectional view of the coupling device of Fig. 1, in a connected but non-tightened state;
Fig. 4 is a cross-sectional view of the coupling device of Fig. 1, in a connected and tightened state;
Fig. 5 is a cross-sectional view of a coupling device according to a second embodiment of the invention, in a connected but non-tightened state; and
Fig. 6 is a cross-sectional view of the coupling device of Fig. 5, in a connected and tightened state.

### Description of preferred embodiments

In the following detailed description preferred embodiments of the invention will be described. However, it is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention, e.g. the length of the coupling device.

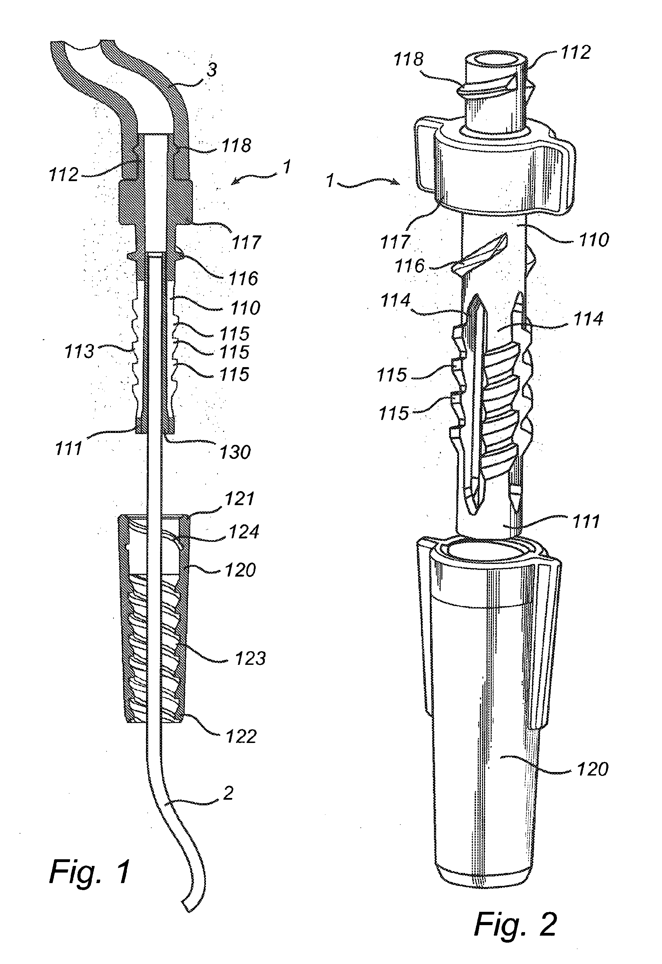

A coupling device according to a first embodiment will first be described with reference to Figs. 1-4. This coupling device 1 for releasable connection of a medical tubing 2, with no coupling parts being inserted into the lumen of the medical tubing comprises a hollow body member 110, compression means in the form of a compression sleeve 120, and a flexible inside tube 130. On the other end, the coupling device is connectable to medical tubing 3, medical devices, or the like.

The hollow body member 110 comprises a first end 111 for receiving a medical tubing end and an opposite second end 112, for conveying an input/output to the medical tubing. At least part of the hollow body member between the first and second end forms an intermediate part 113. The flexible inside tube 130, preferably made of a less rigid material than the hollow body member 120, is preferably arranged inside the inside bore of the hollow body member in such a way that it extends over the entire intermediate part, and is preferably attached to the inside of the hollow body member at least at the ends of the tube.

The intermediate part 113 of this embodiment comprises a plurality of separated fingers 114. The separation distance between the fingers makes it easy for the intermediate part to contract and expand. The separations are preferably open holes, but may also be filled with elastic material or the like (not shown).

The separated fingers each comprises a number of outwardly protruding parts 115, each having a surface tapering in the direction towards the first end of the hollow body member. The protruding parts 115 are arranged in a path forming a screw threading along at least a substantial part of the intermediate part 113.

Between the intermediate part 113 and the second end 112, an engagement area is formed, and provided with a protruding screw thread 116. Between the engagement area and the second end 112, a shoulder portion 117 is further provided.

The second end 112 of the hollow body member may be provided with a protruding screw thread 118 or the like for attachment to conventional medical tubing 3 and/or medical devices. For example, the second end may form a so-called "Luer lock" connection. Such connections are per se well known in the art.

The intermediate part 113 is compressible into a contracted state, in which the inward diameter, facing the medical tubing, is reduced. This contraction is in this embodiment achieved by means of a compression sleeve 120. The compression sleeve forms a collar around part of the hollow body member, and engages with various parts of the hollow body member, as is now to be disclosed in more detail.

The compression sleeve 120 comprises a first end 121 which in use is moved over the first end 111 of the hollow body member, and an opposite second end 122. The inner surface of the internal bore of the compression sleeve is provided with a first screw threading 123, in the vicinity of the second end 122, and a second screw threading 124, in the vicinity of the first end 121. The first screw threading is provided with tapering surface areas, in correspondence with the tapering of the protruding parts 115 of the intermediate part 113 of the hollow body member 110.

The second screw threading 124 of the compression sleeve is arranged to engage with the screw threading 116 of the hollow body member, and the first screw threading 123 of the compression sleeve is arranged to engage with the screw threading formed by the protruding parts 115 of the hollow body member. The screw pitch of the screw threading 124/116 and the screw threading 123/115 is different, and preferably the pitch of the screw threading 124/116 is greater than the pitch of the screw threading 123/115.

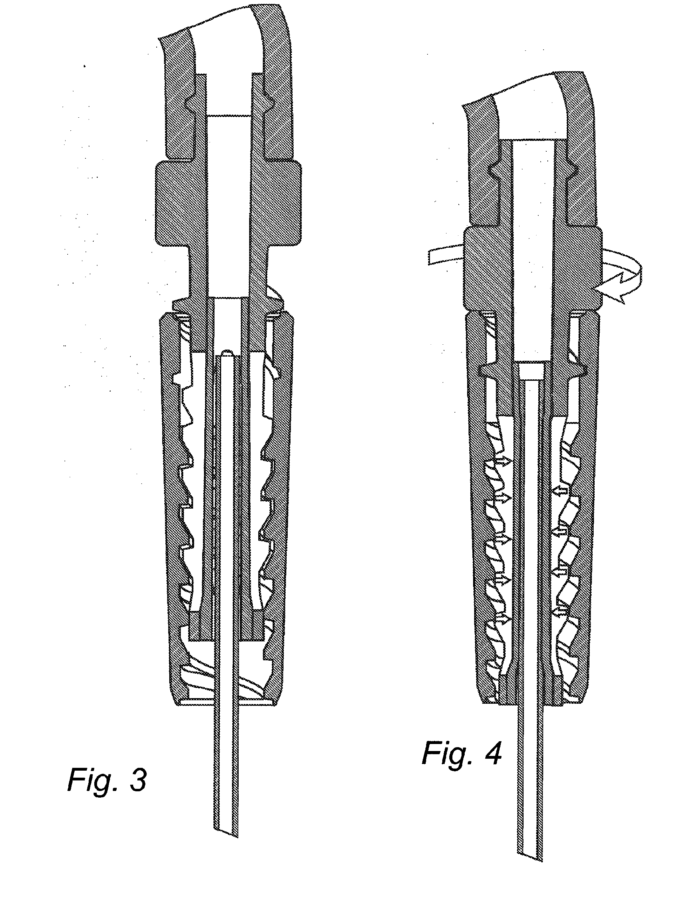

In use, the compression sleeve 120 is arranged in a connected position over the hollow body member 110, and the medical tubing end is inserted through the first end of the hollow body member, and inserted to such an extent that it extends through the intermediate part 113. This state is illustrated in Fig. 3.

Thereafter, the connection between the compression sleeve 120 and the hollow body member 110 is tightened by a relative rotation of the parts. Hereby, the compression sleeve and the hollow body engages primarily in the screw threading 124/116, which forces the screw threading 123 of the compression sleeve to "climb" onto the screw threading 115 of the intermediate part, due to the tapering surfaces, thereby causing the intermediate part to be compressed inwardly. Such a tightened state is illustrated in Fig. 4. In this state, the medical tubing end is connected to the coupling device, and is held firmly in place, without and damage of the medical tubing or the like.

In the tightened position, the shoulder 117 of the hollow body member provides a positive stop in combination with the leading edge 121 of the compression sleeve. Hereby, over-tightening of the connection is prohibited.

A second embodiment will now be discussed. The coupling device of this embodiment generally resembles the first embodiment discussed in the foregoing, but with the following differences related to the intermediate part of the hollow body member.

In this embodiment, the intermediate part 113' comprises, instead of a plurality of protruding parts, each having a tapering surface, only one conically tapering surface 115', extending over more or less the entire intermediate part 113'. The compression sleeve is provided with a corresponding single conically tapering surface 123'. If an elastic material is used, the intermediate part 113' need not be arranged in separated fingers, as in the previously discussed embodiment. However, it is naturally feasible to provide such separate fingers also in this embodiment, whereby the conically tapering part would then consequently be divided into a corresponding number of segments.

In this embodiment, engagement of the compression sleeve and the hollow body member, and subsequent compression and contraction of the intermediate part 113' occurs in a similar way as discussed above in relation to the first embodiment.

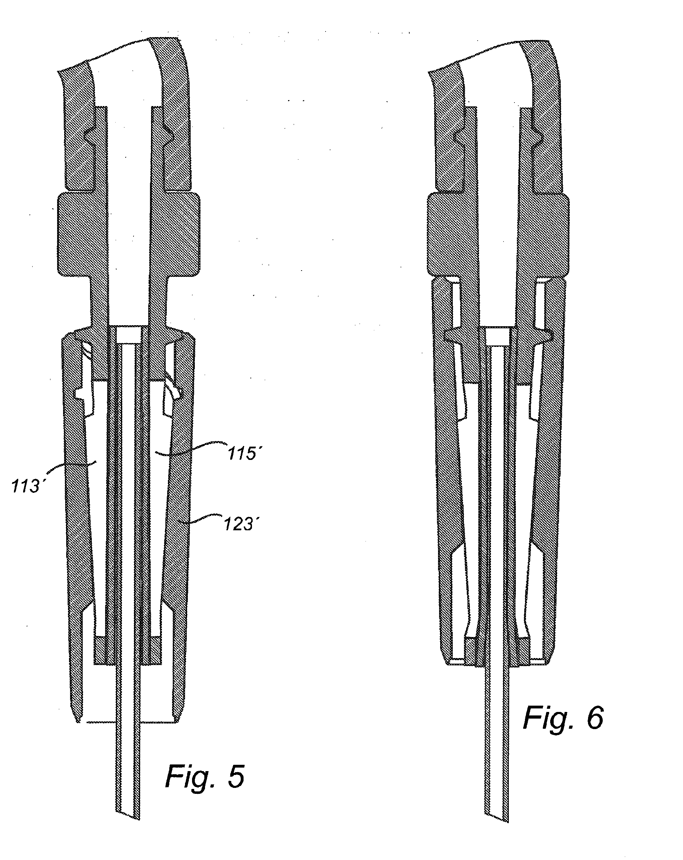

Fig. 5 illustrates a non-tightened state, whereas Fig. 6 illustrates a tightened state.

The hollow body member and the compression sleeve may e.g. be injection molded of a plastic material. These parts are preferably made of a relatively rigid material. The flexible tube may be a piece of ordinary medical tubing, and is preferably made of a somewhat less rigid material. The flexible tube may be connected to the hollow body by means of adhesion, welding or the like. Alternatively, the flexible tube may be integrated with the hollow body. For example, it is possible to produce the hollow body and the flexible tube in a single manufacturing step, by means of two component injection molding or the like.

## Claims

1. A coupling device (1) for releasable connection of a medical tubing (2), such as a catheter, with no coupling parts being inserted into the lumen of the medical tubing, said coupling device comprising:
a hollow body member (110) having a first end (111) for receiving an end part of the medical tubing (2), an opposite second end (112), for conveying an input or output to said medical tubing, and an intermediate part (113; 113') between said first end (111) and second end (112), said intermediate part (113; 113') being arranged to accommodate the end part of the medical tubing (2) inserted through said first end (111) and having an inwardly variable diameter; and
a hollow compression sleeve (120) being arrangeable over the intermediate part of the hollow body member, and being engageable with an engagement area of said hollow body member, whereby engagement between the hollow compression sleeve and the hollow body member forces the hollow compression sleeve to exert a force towards the intermediate part, thereby causing a relatively uniform reduction of the inward diameter of the intermediate part;
wherein the intermediate part (113; 113') of the hollow body member (110) comprises at least one surface tapering in the direction of the first end, and the hollow compression sleeve (120) comprises a corresponding at least one tapering surface, whereby movement of the hollow compression sleeve (120) over said hollow body member (110) in a direction from said first end (111) to said second end (112) provides an engagement between said tapering surfaces, and thereby a compressing force on said intermediate part (113; 113'); and
wherein the engagement between the hollow compression sleeve (120) and the hollow body member (110) occurs through a screw threading (116, 124);
**characterized in that** said engagement area comprising said screw threading (116) in said hollow body member (110) is arranged between the intermediate part (113; 113') and the second end (112).

2. The coupling device of claim 1, wherein the relatively uniform reduction of the inward diameter of the intermediate part (113; 113') is arranged to occur over a substantial length, said length being at least two times the outer diameter of the medical tubing (2).

3. The coupling device of any one of the preceding claims, wherein the intermediate part (113) comprises a plurality of outwardly protruding parts (115; 115'), said parts forming a plurality of tapering surfaces.

4. The coupling device of claim 3, wherein the plurality of protruding parts (115) are formed as a screw threading, and whereby the at least one tapering surface of the hollow compression sleeve (120) formes a corresponding screw threading (123).

5. The coupling device of claim 4, wherein the screw pitch of the screw threading (116, 124) between the hollow compression sleeve and the engagement area of the hollow body member is greater than the screw pitch of the screw threading (115, 123) between hollow compression sleeve and the intermediate part of the hollow body member.

6. The coupling device of any one of the preceding claims, wherein the hollow body member (110) is provided with a shoulder (117), said shoulder forming a positive stop for said hollow compression sleeve (120), prohibiting continued engagement between the hollow compression sleeve (120) and the hollow body member (110) at a certain point, and thereby providing a maximum for the reduction of the inward diameter of the intermediate part (113; 113').

7. The coupling device of any one of the preceding claims, wherein said relatively uniform reduction of the inward diameter of the intermediate part (113; 113') is arranged to occur over a length being at least three times the outer diameter of the medical tubing, and preferably at least five times said diameter.

8. The coupling device of any one of the preceding claims, wherein the intermediate part (113; 113') comprises a plurality of fingers (114), said fingers being to at least some extent spaced apart in a circumferential direction.

9. The coupling device of any one of the preceding claims, further comprising a tube (130) of a flexible material, arranged in the hollow body member (110) in the area of the intermediate part (113; 113'), whereby the tube is situated between the intermediate part (113; 113') of the body member and a medical tubing end inserted into the coupling device.

10. The coupling device of any one of the preceding claims, further comprising a snap locking arrangement automatically locking the hollow compression sleeve on the hollow body member in an engaged position.

## Patentansprüche

1. Kopplungsvorrichtung (1) zur lösbaren Verbindung eines medizinischen Schlauchs (2), wie etwa eines Katheters, ohne dass Kopplungsteile in das Lumen des medizinischen Schlauchs eingefügt werden, wobei die Kopplungsvorrichtung Folgendes umfasst:
ein Hohlkörperelement (110), das ein erstes Ende (111), um ein Endteil des medizinischen Schlauchs (2) aufzunehmen, ein gegenüberliegendes zweites Ende (112), um dem medizinischen Schlauch einen Eingang oder Ausgang zu vermitteln, und ein mittleres Teil (113; 113') zwischen dem ersten Ende (111) und dem zweiten Ende (112) aufweist, wobei das mittlere Teil (113; 113') angeordnet ist,
um das Endteil des medizinischen Schlauchs (2) aufzunehmen, der durch dieses erste Ende (111) eingefügt wird und einen nach innen variablen Durchmesser aufweist; und
eine hohle Kompressionshülse (120), die über dem mittleren Teil des Hohlkörperelements angeordnet werden kann und mit einem Eingriffsbereich des Hohlkörperelements in Eingriff gebracht werden kann, wodurch der Eingriff zwischen der hohlen Kompressionshülse und dem Hohlkörperelement die hohle Kompressionshülse zwingt,
eine Kraft in Richtung auf das mittlere Teil auszuüben, wodurch eine relativ einheitliche Reduzierung des Innendurchmessers des mittleren Teils bewirkt wird;
wobei das mittlere Teil (113; 113') des Hohlkörperelements (110) mindestens eine Oberfläche umfasst, die sich in Richtung auf das erste Ende verjüngt, und die hohle Kompressionshülse (120) eine entsprechende mindestens eine sich verjüngende Oberfläche umfasst,
wodurch die Bewegung der hohlen Kompressionshülse (120) über das Hohlkörperelement (110) in einer Richtung von dem ersten Ende (111) zu dem zweiten Ende (112) einen Eingriff zwischen den sich verjüngenden Oberflächen und dadurch eine Kompressionskraft auf das mittlere Teil (113; 113') bereitstellt; und
wobei der Eingriff zwischen der hohlen Kompressionshülse (120) und
dem Hohlkörperelement (110) durch ein Schraubengewinde (116, 124) erfolgt;
**dadurch gekennzeichnet, dass** der Eingriffsbereich, der das Schraubengewinde (116) in dem Hohlkörperelement (110) umfasst,
zwischen dem mittleren Teil (113; 113') und dem zweiten Ende (112) angeordnet ist.

2. Kopplungsvorrichtung nach Anspruch 1, wobei die relativ einheitliche Reduzierung des Innendurchmessers des mittleren Teils (113; 113') angeordnet ist, um über eine wesentliche Länge zu erfolgen, wobei die Länge mindestens zweimal so groß ist wie der Außendurchmesser des medizinischen Schlauchs (2).

3. Kopplungsvorrichtung nach einem der vorhergehenden Ansprüche, wobei das mittlere Teil (113) eine Vielzahl von nach außen vorstehenden Teilen (115; 115') umfasst, wobei die Teile eine Vielzahl von sich verjüngenden Oberflächen bilden.

4. Kopplungsvorrichtung nach Anspruch 3, wobei die Vielzahl der vorstehenden Teile (115) als Schraubengewinde gebildet ist, und wodurch die mindestens eine sich verjüngende Oberfläche der hohlen Kompressionshülse (120) ein entsprechendes Schraubengewinde (123) bildet.

5. Kopplungsvorrichtung nach Anspruch 4, wobei die Gewindesteigung des Schraubengewindes (116, 124) zwischen der hohlen Kompressionshülse und dem Eingriffsbereich des Hohlkörperelements größer als die Gewindesteigung des Schraubengewindes (115, 123) zwischen der hohlen Kompressionshülse und dem mittleren Teil des Hohlkörperelements ist.

6. Kopplungsvorrichtung nach einem der vorhergehenden Ansprüche, wobei das hohle Körperelement (110) mit einer Schulter (117) versehen ist, wobei die Schulter einen positiven Anschlag für die hohle Kompressionshülse (120) bildet, die einen weitergehenden Eingriff zwischen der hohlen Kompressionshülse (120) und dem Hohlkörperelement (110) an einem gewissen Punkt untersagt und dadurch ein Maximum für die Reduzierung des Innendurchmessers des mittleren Teils (113; 113') bereitstellt.

7. Kopplungsvorrichtung nach einem der vorhergehenden Ansprüche, wobei die relativ einheitliche Reduzierung des Innendurchmessers des mittleren Teils (113; 113') angeordnet ist, um über eine Länge zu erfolgen, die mindestens dreimal so groß ist wie der Außendurchmesser des medizinischen Schlauchs und bevorzugt mindestens fünfmal so groß ist wie dieser Durchmesser.

8. Kopplungsvorrichtung nach einem der vorhergehenden Ansprüche, wobei das mittlere Teil (113; 113') eine Vielzahl von Fingern (114) umfasst, wobei die Finger mindestens einigermaßen in einer Umfangsrichtung beabstandet sind.

9. Kopplungsvorrichtung nach einem der vorhergehenden Ansprüche, ferner umfassend eine Röhre (130) aus einem biegsamen Material, die in dem Hohlkörperelement (110) in dem Bereich des mittleren Teils (113; 113') angeordnet ist, wodurch sich die Röhre zwischen dem mittleren Teil (113; 113') des Körperelements und einem medizinischen Schlauchende, das in die Kopplungsvorrichtung eingefügt ist, befindet.

10. Kopplungsvorrichtung nach einem der vorhergehenden Ansprüche, ferner umfassend eine Schnappverschlussanordnung, welche die hohle Kompressionshülse auf dem Hohlkörperelement in einer Eingriffsposition feststellt.

## Revendications

1. Dispositif de couplage (1) pour une connexion amovible d'une tubulure médicale (2) comme un cathéter, aucune partie de couplage n'étant insérée dans le lumen de la tubulure médicale, ledit dispositif de couplage comprenant :
un élément de corps creux (110) ayant une première extrémité (111) pour recevoir une partie d'extrémité de la tubulure médicale (2), une seconde extrémité opposée (112), pour transporter une entrée ou une sortie vers ladite tubulure médicale, et une partie intermédiaire (113 ; 113') entre ladite première extrémité (111) et la seconde extrémité (112), ladite partie intermédiaire (113 ; 113') étant disposée pour loger la partie d'extrémité de la tubulure médicale (2) insérée par ladite première extrémité (111) et ayant un diamètre variable intérieurement ; et
un manchon de compression creux (120) pouvant être disposé sur la partie intermédiaire de l'élément de corps creux, et pouvant être enclenché avec une zone d'enclenchement dudit élément de corps creux, par lequel l'enclenchement entre le manchon de compression creux et l'élément de corps creux force le manchon de compression creux à exercer une force vers la partie intermédiaire, entraînant ainsi une réduction relativement uniforme du diamètre intérieur de la partie intermédiaire ;
dans lequel la partir intermédiaire (113; 113') de l'élément de corps creux (110) comprend au moins une surface se réduisant dans le sens de la première extrémité, et le manchon de compression creux (120) comprend une au moins surface décroissante correspondante, tandis que le déplacement du manchon de compression creux (120) sur ledit élément de corps creux (110) dans un sens allant de ladite première extrémité (111) vers ladite seconde extrémité (112) assure un enclenchement entre lesdites surfaces décroissantes, et ainsi une force de compression sur ladite partie intermédiaire (113; 113') ; et
dans lequel l'enclenchement entre le manchon de compression creux (120) et l'élément de corps creux (110) se produit par un filetage de vis (116, 124) ;
**caractérisé en ce que** ladite zone d'enclenchement comprenant ledit filetage de vis (116) dans le dit élément de corps creux (110) est disposée entre la partie intermédiaire (113 ; 113') et la seconde extrémité (120).

2. Dispositif de couplage de la revendication 1, dans lequel la réduction relativement uniforme du diamètre intérieur de la partie intermédiaire (113 ; 113') est arrangée de manière à se produire sur une longueur substantielle, ladite longueur étant au moins deux fois le diamètre extérieur de la tubulure médicale (2).

3. Dispositif de couplage selon l'une quelconque des revendications précédentes, dans lequel la partie intermédiaire (113) comprend une pluralité de parties dépassant vers l'extérieur (115 ; 115'), lesdites parties formant une pluralité de surfaces décroissantes.

4. Dispositif de couplage de la revendication 3, dans lequel la pluralité de parties dépassant (115) est formée comme un filetage de vis, et par lequel la au moins une surface décroissante du manchon de compression creux (120) forme un filetage de vis correspondant (123).

5. Dispositif de couplage de la revendication 4, dans lequel le pas de vis du filetage de vis (116, 124) entre le manchon de compression creux et la zone d'enclenchement de l'élément de corps creux est plus grand que le pas de vis du filetage de vis (115, 123) entre le manchon de compression creux et la partie intermédiaire de l'élément de corps creux.

6. Dispositif de couplage selon l'une quelconque des revendications précédentes, dans lequel l'élément de corps creux (110) est muni d'un épaulement (117) formant un arrêt positif pour ledit manchon de compression creux (120), empêchant l'enclenchement continu entre ledit manchon de compression creux (120) et l'élément de corps creux (110) à un certain point, et fournissant ainsi un maximum pour la réduction du diamètre intérieur de la partie intermédiaire (113 ; 113').

7. Dispositif de couplage selon l'une quelconque des revendications précédentes, dans lequel la réduction relativement uniforme du diamètre intérieur de la partie intermédiaire (113 ; 113') est arrangée de manière à se produire sur une longueur étant au moins trois fois le diamètre extérieur de la tubulure médicale et préférablement d'au moins cinq fois ledit diamètre.

8. Dispositif de couplage selon l'une quelconque des revendications précédentes, dans lequel la partie intermédiaire (113 ; 113') comprend une pluralité de doigts (114), lesdits doigts étant au moins dans une certaine mesure espacés dans une direction circonférentielle.

9. Dispositif de couplage selon l'une quelconque des revendications précédentes, comprenant en outre un tube (130) d'un matériau flexible, disposé dans l'élément de corps creux (110) dans la zone de la partie intermédiaire (113 ; 113'), par lequel le tube est situé entre la partie intermédiaire (113 ; 113') de l'élément de corps et une extrémité de tubulure médicale insérée dans le dispositif de couplage.

10. Dispositif de couplage selon l'une quelconque des revendications précédentes, comprenant en outre un verrou d'accrochage verrouillant automatiquement le manchon de compression creux sur l'élément de corps creux dans une position enclenchée.
